# FASCICULE DE BREVET EUROPEEN

(11) **EP 0 509 885 B1**
(45) Date de publication et mention de la délivrance du brevet: **19.06.1996**
(21) Numéro de dépôt: 92401005.1
(22) Date de dépôt: 09.04.1992
(51) Int. Cl.: C01B 7/19, C07C 17/38, C07C 19/08

(54) **Procédé de séparation du fluorure d'hydrogène de ses mélanges avec le 1,1,1-trifluoro-2-chloroéthane**
Verfahren zum Abtrennen von Wasserstoffluoriden aus ihren Gemischen mit 1,1,1-Trifluoro-2-Chlorethan
Process for separating hydrogen fluoride from its mixtures with 1,1,1-trifluoro-2-chloroethane

(30) Priorité: 17.04.1991 FR 9104736
(43) Date de publication de la demande: 21.10.1992
(73) Titulaire: ELF ATOCHEM S.A., F-92800 Puteaux (FR)
(72) Inventeur: Berthe, Bernard, F-69110 Sainte Foy les Lyon (FR)
(74) Mandataire: Leboulenger, Jean

(56) Documents cités:
- EP-A- 0 098 341
- EP-A- 0 353 970
- EP-A- 0 354 697

## Description

L'invention concerne la séparation du fluorure d'hydrogène (HF) de ses mélanges avec le 1,1,1-trifluoro-2-chloroéthane (F133a) qui est un important intermédiaire de synthèse, utilisable notamment pour la fabrication du 1,1,1,2-tétrafluoroéthane (F134a).

Le procédé selon l'invention s'applique plus particulièrement à la séparation de l'HF non transformé contenu dans les mélanges provenant de la fabrication du F133a par fluoration du trichloroéthylène ou du tétrachloroéthane symétrique ou asymétrique. Pour des raisons économiques, il est nécessaire de récupérer l'HF sous forme anhydre afin de pouvoir le recycler au réacteur de fluoration.

Diverses techniques pour effectuer la séparation d'HF et d'hydrocarbures chlorofluorés ont déjà été décrites. On peut citer par exemple :
- le brevet US 2 640 086 qui concerne la séparation de l'HF et du chlorodifluorométhane et utilise du chloroforme pour favoriser la décantation en deux phases, une phase riche en HF et une phase pauvre en HF ;
- le brevet US 3 873 629 relatif à un procédé continu pour la séparation d'HF et de chlorodifluorométhane et consistant à mettre en contact à contre-courant le mélange gazeux des deux constituants avec de l'acide sulfurique ;
- le brevet US 3 976 447 qui propose une séparation de l'HF d'effluents gazeux par absorption-désorption sur des particules de chlorure de calcium, baryum ou strontium ;
- le brevet US 4 209 470 qui décrit un procédé de séparation de l'HF de ses mélanges avec le 1-chloro-1,1-difluoroéthane dans lequel, pour améliorer la décantation, on ajoute un liquide auxiliaire constitué totalement ou majoritairement par du 1,1-dichloro-1-fluoroéthane ;
- la demande de brevet EP 0 353 970 relative à la séparation d'HF de ses mélanges avec le 2,2-dichloro-1,1,1-trifluoroéthane et/ou le 2-chloro-1,1,1,2-tétrafluoroéthane par décantation et distillation;
- la demande de brevet EP 0 098 341 relatif à la séparation d'HF de ses mélanges avec le 1-chloro-1,1-difluoroéthane.

Dans le cas des mélanges d'HF et de F133a, une simple distillation ne permet pas de les séparer car l'HF et le F133a forment un azéotrope plus volatil que l'HF ou le F133a ; la teneur en HF de cet azéotrope est d'environ 60 % molaire (20 % en poids). Il n'existe dans la littérature aucune donnée sur la décantation des mélanges d'HF et de F133a ; à température ambiante et quelles que soient les concentrations en HF et F133a, les mélanges d'HF et de F133a ne décantent pas en deux phases.

Il a maintenant été trouvé qu'on peut obtenir une excellente séparation d'un mélange d'HF et de F133a à condition de le refroidir à une température inférieure à 0°C, de préférence comprise entre -40°C et -10°C. Ainsi, par exemple à -20°C, la décantation du mélange azéotropique HF-F133a fournit une phase inférieure organique ne contenant que 2,7 % en poids d'HF (14 % molaire) et une phase supérieure acide contenant 60 % en poids d'HF (90 % molaire). Dès lors, en combinant décantation et distillation, il s'est avéré possible d'obtenir une séparation éventuellement complète de l'HF et du F133a.

Le procédé de séparation d'HF et de F133a selon l'invention est donc caractérisé en ce que :
**a)** le mélange d'HF et de F133a est soumis à une décantation à une température inférieure à 0°C,
**b)** la phase inférieure organique pauvre en HF ainsi obtenue est distillée de façon à séparer en tête l'HF contenu dans cette phase, sous forme d'azéotrope HF-F133a qui est renvoyé au décanteur et à récupérer en pied le F133a excédentaire, et
**c)** la phase supérieure riche en HF est soit recyclée directement au réacteur de fluoration, soit soumise à une distillation de façon à séparer en tête le F133a contenu dans cette phase, sous forme d'azéotrope HF-F133a qui est renvoyé au décanteur et à récupérer en pied l'HF pratiquement pur.

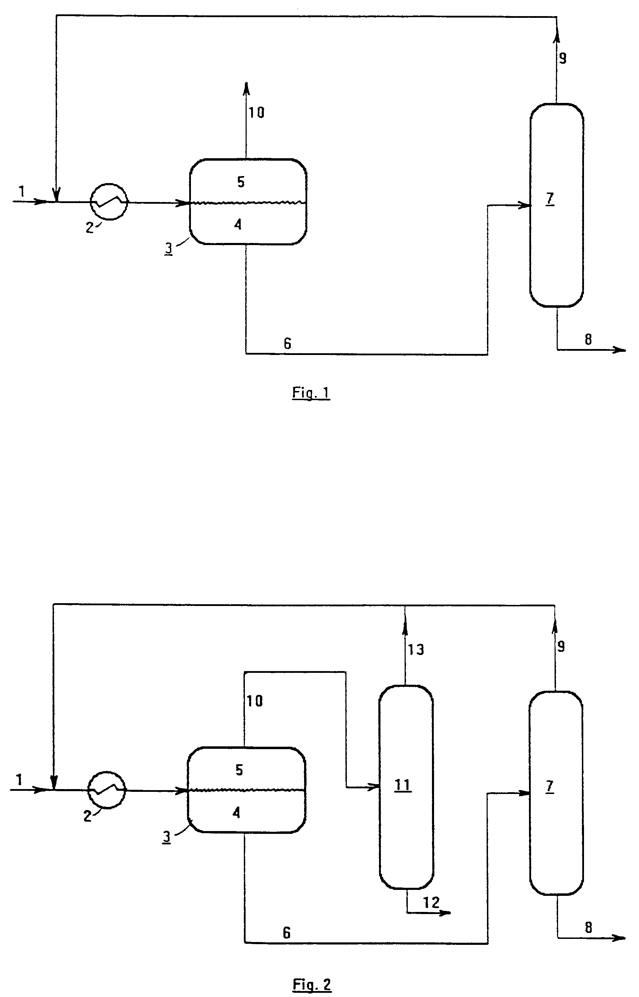

Le fonctionnement du procédé selon l'invention sera mieux compris en se référant aux schémas représentés sur les figures 1 et 2 annexées.

Le mélange à séparer, constitué essentiellement d'HF et de F133a, préalablement refroidi par l'intermédiaire d'un échangeur 2, est alimenté par la conduite 1 dans le décanteur 3 maintenu à une température inférieure à 0°C, de préférence comprise entre -40°C et -10°C. Par démixion, on obtient alors dans le décanteur une phase inférieure organique 4, pauvre en HF, et une phase supérieure 5, riche en HF. La phase organique 4 issue du décanteur 3 alimente par 6 une colonne à distiller 7 au sommet de laquelle on sort un effluent 9 de composition azéotropique en HF et F133a ; cet effluent 9 est renvoyé au décanteur 3 en amont de l'échangeur 2 pour séparation en deux phases. En pied de la colonne 7, on récupère un flux 8 de F133a pur.

La phase supérieure 5, riche en HF, sortant par la conduite 10 peut être recyclée telle quelle directement au réacteur de fluoration pour la production de F133a (figure 1). Cependant, suivant la figure 2 correspondant au mode de réalisation préféré du procédé selon l'invention, la phase supérieure 5, riche en HF, est amenée par la conduite 10 à une colonne à distiller 11, en tête de laquelle on sort un effluent 13 de composition azéotropique en HF et F133a qui, comme l'effluent 9, est renvoyé au décanteur 3 en amont de l'échangeur 2 pour séparation en deux phases. En pied de la colonne 11, on récupère alors en 12 un HF pratiquement pur.

Le passage du décanteur aux colonnes à distiller et de celles-ci vers le décanteur se fait par l'intermédiaire de vannes de détente ou de pompes selon les pressions de fonctionnement du décanteur et des colonnes à distiller. La température des flux alimentant par 6 et 10 les colonnes à distiller peut être ajustée par l'intermédiaire d'échangeurs pour obtenir une distillation optimale.

A titre d'exemple, le tableau suivant donne les compositions molaires, températures et pressions des différents flux obtenus en procédant conformément au schéma de la figure 2 à partir d'un mélange (1) d'HF et de F133a comprenant de 14 à 90 % en moles d'HF et de 10 à 86 % en moles de F133a.

**TABLEAU I**

| | Phase organique (4) | Phase HF (5) | Tête des colonnes (9 et 13) | Pied de colonne 7 (8) | Pied de colonne 11 (12) |
|---|---|---|---|---|---|
| HF (% moles) | 14 | 90 | 60 | - | 100 |
| F133a (% moles) | 86 | 10 | 40 | 100 | - |
| Température (°C) | -20 | -20 | +17 | +27 | +43 |
| Pression (bars absolus) | 15 | 15 | 2,2 | 2,2 | 2,2 |

Des essais à différentes pressions (1, 10, 15 et 25 bars) ont montré que ce paramètre n'a pas d'influence notable sur la décantation du mélange d'HF et de F133a. Son influence sur les températures en pied et en tête des colonnes 7 et 11 est illustrée dans les tableaux II et III suivants :

**TABLEAU II**

| | Phase organique (4) | Phase HF (5) | Tête des colonnes (9 et 13) | Pied de colonne 7 (8) | Pied de colonne 11 (12) |
|---|---|---|---|---|---|
| HF (% moles) | 14 | 90 | 60 | - | 100 |
| F133a (% moles) | 86 | 10 | 40 | 100 | - |
| Température (°C) | -20 | -20 | +42 | +54 | +70 |
| Pression (bars absolus) | 15 | 15 | 5 | 5 | 5 |

**TABLEAU III**

| | Phase organique (4) | Phase HF (5) | Tête des colonnes (9 et 13) | Pied de colonne 7 (8) | Pied de colonne 11 (12) |
|---|---|---|---|---|---|
| HF (% moles) | 14 | 90 | 60 | - | 100 |
| F133a (% moles) | 86 | 10 | 40 | 100 | - |
| Température (°C) | -20 | -20 | +66 | +81 | +96 |
| Pression (bars absolus) | 15 | 15 | 10 | 10 | 10 |

La température est le paramètre essentiel pour la décantation. En effet, au-dessus de 15°C, on n'observe aucune décantation quelle que soit la composition du mélange HF-F133a. A -20°C, la phase HF décantée contient 60 % en poids d'HF ; cette teneur passe à 50 % pour une température de décantation de -5°C. A cette dernière température, la phase organique décantée contient 5 % en poids d'HF ; cette teneur passe à 2,7 % lorsque la décantation est effectuée à -20°C. Une bonne maîtrise de la température au décanteur est donc importante pour obtenir une décantation optimale.

Dans le mélange d'HF et de F133a alimenté au décanteur, la teneur en HF peut aller de 14 à 90 % en mole ; elle est le plus souvent comprise entre 25 et 75 %.

Lorsque le F133a est préparé par fluoration du trichloroéthylène ou d'un tétrachloroéthane, les effluents du réacteur de fluoration contiennent généralement, en plus du F133a et de l'HF non converti, de l'acide chlorhydrique qui peut être éliminé facilement par distillation, et une petite proportion (jusqu'à 20 % en poids par rapport au F133a) d'autres composés organiques tels que, par exemple, le trichloroéthylène, le monofluorotrichloroéthane, le difluorodichloroéthane. Des essais de décantation ont montré que la présence de ces composés organiques ne nuit en aucun cas à la décantation et peut même la favoriser. A titre d'exemple, le tableau IV suivant indique les flux molaires obtenus en procédant conformément au schéma de la figure 2 à partir de l'effluent d'un réacteur de fluoration du trichloroéthylène en F133a. Cet effluent qui contient un peu de 1,1-difluoro-1,2-dichloroéthane (F132b) est préalablement distillé pour éliminer l'acide chlorhydrique sous-produit et alimente le décanteur 3 par la conduite 1. Cet effluent est refroidi à -20°C par l'échangeur 2 et le décanteur est maintenu à -20°C.

**TABLEAU IV**

| | **DEBITS EN MOLES/HEURE :** | | |
|---|---|---|---|
| | HF | F133a | F132b |
| Alimentation (1) | 40 | 19 | 1 |
| Phase organique (4) | 2,7 | 20,8 | 0,94 |
| Phase HF (5) | 45,7 | 3,8 | 0,06 |
| Tête de colonne 11 (13) | 5,7 | 3,8 | - |
| Tête de colonne 7 (9) | 2,7 | 1,8 | - |
| Pied de colonne 11 (12) | 40 | - | 0,06 |
| Pied de colonne 7 (8) | - | 19 | 0,94 |

Le pied de la colonne 11, contenant principalement de l'HF et des traces de F132b, peut être renvoyé tel quel au réacteur de fluoration.

Le pied de la colonne 7, contenant le F133a et la majeure partie du F132b alimenté au décanteur, est ensuite distillé pour obtenir le F133a pur en tête de colonne. Le pied de cette colonne à distiller, très riche en F132b, est alors renvoyé au réacteur de fluoration.

On constate que la teneur molaire en HF de la phase organique (4) ainsi obtenue n'est que de 11 %, à comparer à celle (14 %) obtenue en l'absence de F132b.

Le procédé de séparation selon l'invention s'applique donc non seulement aux mélanges binaires HF-F133a, mais aussi aux mélanges bruts de fluoration, après élimination du HCl sous-produit.

## Revendications

1. Procédé de séparation du fluorure d'hydrogène (HF) de ses mélanges avec le 1,1,1-trifluoro-2-chloroéthane (F133a), caractérisé en ce que :
**a)** le mélange d'HF et de F133a est soumis à une décantation à une température inférieure à 0°C,
**b)** la phase inférieure organique pauvre en HF ainsi obtenue est distillée de façon à séparer en tête l'HF contenu dans cette phase, sous forme d'azéotrope HF-F133a qui est renvoyé au décanteur et à récupérer en pied le F133a excédentaire, et
**c)** la phase supérieure riche en HF est soit recyclée directement au réacteur de fluoration, soit soumise à une distillation de façon à séparer en tête le F133a contenu dans cette phase, sous forme d'azéotrope HF-F133a qui est renvoyé au décanteur et à récupérer en pied du HF pratiquement pur.

2. Procédé selon la revendication 1, dans lequel la température de décantation est comprise entre -40°C et -10°C.

3. Procédé selon la revendication 1 ou 2, dans lequel la teneur en HF du mélange à traiter est comprise entre 14 et 90 % en moles, de préférence entre 25 et 75 %.

4. Procédé selon l'une des revendications 1 à 3, dans lequel le mélange d'HF et de F133a à traiter contient en outre jusqu'à 20 % en poids d'autres composés organiques (% exprimé par rapport au F133a).

## Claims

1. Process for separating hydrogen fluoride (HF) from its mixtures with 1,1,1-trifluoro-2-chloroethane (F133a), characterised in that:
a) the mixture of HF and F133a is subjected to a phase separation at a temperature below 0°C,
b) the HF-poor organic lower phase thus obtained is distilled so as to separate off at the head the HF present in this phase, in the form of HF-F133a azeotrope which is returned to the phase separator, and to recover the excess F133a at the foot, and
c) the HF-rich upper phase is either recycled directly to the fluorination reactor or is subjected to a distillation so as to separate at the head the F133a present in this phase, in the form of HF-F133a azeotrope which is returned to the phase separator, and to recover practically pure HF at the foot.

2. Process according to Claim 1, in which the temperature of phase separation is between -40°C and -10°C.

3. Process according to Claim 1 or 2, in which the HF content of the mixture to be treated is between 14 and 90 mol%, preferably between 25 and 75 %.

4. Process according to one of Claims 1 to 3, in which the mixture of HF and F133a to be treated also contains up to 20 % by weight of other organic compounds (% expressed relative to F133a).

## Patentansprüche

1. Verfahren zur Abtrennung von Fluorwasserstoff (HF) von seinen Mischungen mit 1,1,1-Trifluor-2-chlorethan (F133a), dadurch gekennzeichnet, daß
a) die Mischung aus HF und F133a einer Dekantierung bei einer Temperatur unterhalb von 0 °C unterworfen wird,
b) die so erhaltene, an HF arme, untere organische Phase destilliert wird, um am Kolonnenkopf in dieser Phase enthaltenes HF in Form eines HF/F133a-Azeotrops abzutrennen, das wieder der Dekantiervorrichtung zugeführt wird, und um am Kolonnenfuß überschüssiges F133a zurückzugewinnen, und
c) die an HF reiche, obere Phase entweder direkt im Fluorierungsreaktor wieder eingesetzt wird oder einer Destillation unterworfen wird, um am Kolonnenkopf das in dieser Phase enthaltene F133a in Form eines HF/F133a-Azeotrops abzutrennen, das wieder der Dekantiervorrichtung zugeführt wird, und um am Kolonnenfuß praktisch reines HF zurückzugewinnen.

2. Verfahren nach Anspruch 1, bei dem die Dekantierungstemperatur zwischen -40 °C und -10 °C liegt.

3. Verfahren nach Anspruch 1 oder 2, bei dem der HF-Gehalt der zu behandelnden Mischung zwischen 14 und 90 Mol-%, vorzugsweise zwischen 25 und 75 Mol-%, liegt.

4. Verfahren nach einem der Ansprüche 1 bis 3, bei dem die zu behandelnde HF/F133a-Mischung zusätzlich bis zu 20 Gew.-% weitere organische Verbindungen enthält, wobei die Prozentangaben sich auf F133a beziehen.
